# EUROPÄISCHE PATENTSCHRIFT

(11) **EP 0 869 951 B1**
(45) Veröffentlichungstag und Bekanntmachung des Hinweises auf die Patenterteilung: **12.09.2001**
(21) Anmeldenummer: 96943080.0
(22) Anmeldetag: 11.12.1996
(51) Int. Cl.: C07D 303/22, C07C 29/17, C07C 29/132

(54) **VERFAHREN ZUR HERSTELLUNG VON 1,6-HEXANDIOL AUS EPOXIBUTADIEN**
METHOD OF PRODUCING 1,6-HEXANE DIOL FROM EPOXYBUTADIENE
PROCEDE DE PRODUCTION DE 1,6-HEXANDIOL A PARTIR D'EPOXYBUTADIENE

(30) Priorität: 18.12.1995 DE 19547213
(43) Veröffentlichungstag der Anmeldung: 14.10.1998
(73) Patentinhaber: BASF AKTIENGESELLSCHAFT, 67056 Ludwigshafen (DE)
(72) Erfinder: FISCHER, Rolf, D-69121 Heidelberg (DE); KRATZ, Detlef, D-69121 Heidelberg (DE); PINKOS, Rolf, D-67098 Bad Dürkheim (DE); SCHÄFER, Martin, D-67063 Ludwigshafen (DE); HÖHN, Arthur, D-67281 Kirchheim (DE)
(86) Internationale Anmeldenummer: EP9605524
(87) Internationale Veröffentlichungsnummer: WO9722601

(56) Entgegenhaltungen:
- US-A- 2 561 984
- US-A- 3 070 633
- CARBOHYDRATE RESEARCH, Bd. 83, 1980, AMSTERDAM, NL, Seiten 63-72, XP002028911 J. KUSZMANN, ET AL.: "Synthesis of 1,2:5,6-dianhydro-3,4-dideoxy-erythro- and -D-threo-hexitol and their E-3-ene derivatives" in der Anmeldung erwähnt
- PATENT ABSTRACTS OF JAPAN vol. 002, no. 008 (C-001), 20.Januar 1978 & JP 52 108940 A (TOYO SODA MFG CO LTD), 12.September 1977,

## Beschreibung

Die vorliegende Erfindung betrifft ein neues Verfahren zur Herstellung von 1,6-Hexandiol aus Epoxibutadien.

1,6-Hexandiol ist ein bedeutendes Zwischenprodukt für die Herstellung von Polyestern und Polyurethanen. Die US-A-3,070,633 beschreibt ein Verfahren zur Herstellung von 1,6-Hexandiol durch Hydrierung von 2,5-Tetrahydrofurandimethanol. Im technischen Maßstab wird es durch Hydrierung von Adipinsäure oder deren Derivaten hergestellt (Weissermel, Arpe, Industrielle Organische Chemie, 4. Auflage, Verlag Chemie 1994, S. 263). Nachteilig an diesen Verfahren ist die Verwendung korrosiver Stoffströme, die teure Werkstoffe erfordern. Weiterhin ist die Hydrierung nur im Hochdruckbereich wirtschaftlich, so daß für entsprechende Reaktoren hohe Investitionskosten anfallen. Schließlich erfordert die Hydrierung eines Äquivalents Adipinsäure 4 Äquivalente Wasserstoff.

Die Herstellung von Bisepoxihexatrienen der Formeln Ia und Ib 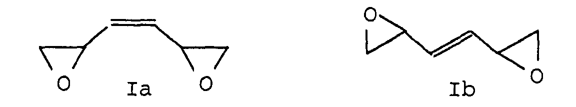 ist nach Kuszmann et al., Carbohydrate Research, 83 (1980) 63 durch Umsetzung von Ditosylaten oder Dimesylaten von Tetrolen mit überstöchiometrischen Mengen Base möglich. Da jedoch die benötigten Vorprodukte nur aufwendig herstellbar sind und die Methode mit dem Anfall stöchiometrischer Mengen Salz verknüpft ist, eignet sie sich nicht für die Herstellung der Bisepoxitriene in großen Mengen.

Es bestand die Aufgabe, ein Verfahren zur Verfügung zu stellen, das die Herstellung von 1,6-Hexandiol bei wesentlich niedrigeren Drücken als nach dem Stand der Technik erlaubt. Weiterhin soll das Verfahren mit weniger Wasserstoff pro Äquivalent Hexandiol auskommen. Ein weiterer Aspekt der Aufgabe bestand darin, ein Verfahren zu finden, das die Herstellung von Bisepoxihexatrienen der Formeln Ia und Ib unter Vermeidung der oben genannten Nachteile des Standes der Technik ermöglicht.

Demgemäß wurde ein Verfahren zur Herstellung von 1,6-Hexandiol gefunden, das dadurch gekennzeichnet ist, daß man
a) Epoxibutadien in Gegenwart eines Metathesekatalysators unter Abspaltung von Ethen zu Bisepoxihexatrienen der Formeln Ia und Ib umsetzt und
b) diese Bisepoxihexatriene mit Wasserstoff zu 1,6-Hexandiol hydriert.

Das Gesamtverfahren zur Herstellung von 1,6-Hexandiol aus Epoxibutadien ist in der folgenden Reaktionsgleichung wiedergegeben:

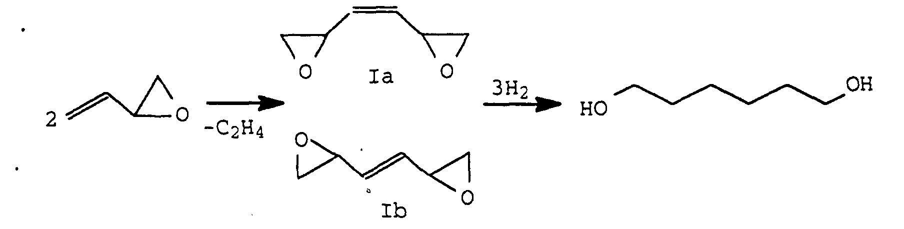

### Verfahrensschritt a)

Erfindungsgemäß werden 2 Äquivalente Epoxibutadien unter Abspaltung von Ethen zu den Bisepoxitrienen der Formeln Ia und Ib umgesetzt. Epoxibutadien kann nach der Lehre der US-A 4 897 498 durch Epoxidation von Butadien hergestellt werden. Es wird bevorzugt in reiner Form eingesetzt, kann jedoch auch Nebenbestandteile wie Crotonaldehyd, 2,3-Dihydrofuran und 2,5-Dihydrofuran enthalten. Die Umsetzung findet in Gegenwart eines Metathesekatalysators statt. Darunter sind Katalysatoren zu verstehen, die ein Gleichgewicht vom Typ 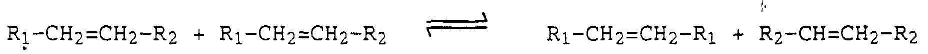 einstellen, wobei R₁ und R₂ für organische Reste stehen. Die Katalysatoren sind an sich bekannt. Es handelt sich um heterogene oder homogene Übergangsmetallverbindungen, insbesondere um solche von Übergangsmetallen der Gruppen 4 sowie 6 bis 10 des Periodischen Systems der Elemente. Solche Katalysatoren sind beispielsweise in Parshall, Ittel, Homgeneous Catalysis, 2. Auflage, Wiley 1992, S. 217-236, weiterhin in Banks, Catalysis, Bd. 4, S. 100-129 und in Grubbs, Progress in Inorg. Chem., Bd. 24, S. 1-50 beschrieben. _

Bevorzugte Metathesekatalysatoren enthalten Ruthenium. Besonders bevorzugte Katalysatoren sind Rutheniumverbindungen der allgemeinen Zusammensetzung RuX₂(=CHR)(PR'₃)₂, wobei X für Halogene wie Fluor, Chlor, Brom und Jod, R für Wasserstoff, Alkyl, Cycloalkyl, Aryl oder Aralkyl und R' für Alkyl, Cycloalkyl, Aryl oder Aralkyl steht und wie sie in der WO-A 93/20111 beschrieben sind. Besonders bevorzugt sind auch Katalysatoren der Zusammensetzung RuX₂(Aren)/PR₃, wobei X für Halogene wie Fluor, Chlor, Brom und Jod und R für Wasserstoff, Alkyl, Cycloalkyl, Aryl oder Aralkyl steht und wie sie - weiterhin auch in Kombination mit einer Diazoalkanverbindung - in Noels, J.Chem.Soc., Chem. Commun. 1995, S. 1127-1128 beschrieben sind. Beispielhaft genannt seien RuCl₂(=CHPh) (PCy₃)₂, RuCl₂(p-cumol)/PCy₃/N₂CHSiMe₃ und RuCl₂(p-cumol)(PCy₃), wobei Ph für Phenyl und Cy für Cyclohexyl steht.

Die Menge des Katalysators, bezogen auf Epoxibutadien, kann in weiten Grenzen variiert werden, z.B. 10⁻⁵ bis 1 mol/mol Epoxibutadien, bevorzugt 10⁻⁴ bis 10⁻¹, besonders bevorzugt 2·10⁻⁴ bis 5·10⁻² mol/mol Epoxibutadien.

Die Umsetzung von Epoxibutadien an den genannten Katalysatoren kann in der Gas- oder der Flüssigphase durchgeführt werden. Die Metathese wird im allgemeinen bei Temperaturen von -20 bis 400°C durchgeführt. Bevorzugt ist die Umsetzung in der Flüssigphase bei Temperaturen von -10 bis 150°C, besonders bevorzugt 15 bis 100°C. Dabei kann der Druck vorteilhaft so gewählt werden, daß zumindest Epoxibutadien flüssig vorliegt.

Die Reaktion kann kontinuierlich oder diskontinuierlich ausgeführt werden.

Das während der Reaktion freigesetzte Ethen wird bevorzugt kontinuierlich aus dem Reaktionsgemisch entfernt. Ethen kann mit einem Inertgas wie Methan, Stickstoff, Argon oder Kohlendioxid ausgetrieben werden. Bevorzugt ist jedoch eine Verfahrensweise, in der Ethen durch den sich einstellenden Eigendampfdruck aus dem Reaktor gelangt.

Die Metathesestufe kann ohne oder in Gegenwart eines Lösungsmittels durchgeführt werden. Als Lösungsmittel kommen beispielsweise in Betracht: Ether wie Tetrahydrofuran, Ethylenglycoldimethylether, Diethylether und Methyl-tert.-butylether, Kohlenwasserstoffe wie Cyclohexan, Benzol, Toluol und Cumol, Halogenkohlenwasserstoffe wie Chloroform, Methylenchlorid, Dichlorethane, Chlorpropane und Chlorbutane sowie Ester wie Essigsäureethylester.

Als Reaktoren kommen für die Metathesereaktion beispielsweise Rührkessel, Schlaufenreaktoren oder Rohrreaktoren in Betracht. Es hat sich als vorteilhaft erwiesen, insbesondere kleine Ansätze unter einer Schutzgasatmosphäre durchzuführen, beispielsweise unter Argon.

Die Metathese ist in der Regel nach 0,5 bis 48 h Reaktionszeit beendet. Das erhaltene Reaktionsgemisch kann direkt einer Hydrierung unterworfen werden. Sollen jedoch die erfindungsgemäß erhaltenen Bisepoxihexatriene isoliert werden, können alle flüchtigen Bestandteile des Reaktionsgemisch destillativ vom Katalysator abgetrennt werden. Der Katalysator verbleibt dabei im Sumpf und kann - gegebenenfalls nach Aufarbeitung - in die Reaktion zurückgeführt werden. Die Bisepoxihexatriene der Formeln Ia und Ib können destillativ von der Ausgangsverbindung abgetrennt werden, welche ebenfalls in die Reaktion zurückgeführt werden kann.

### Verfahrensschritt b)

Die gemäß Verfahrensschritt a) erhaltenen Verbindungen der Formeln Ia und Ib können durch Hydrierung zu 1,6-Hexandiol umgesetzt werden. Dazu kann der Reaktionsaustrag aus der Metathese, sofern der Metathesekatalysator homogen darin vorliegt, direkt ohne Zusatz eines weiteren Hydrierkatalysators mit Wasserstoff hydriert werden. Sollte in der Metathesestufe kein vollständiger Umsatz erreicht worden sein, ist es vorteilhaft, vor der Hydrierung Epoxibutadien destillativ abzutrennen. Verzichtet man auf diese Abtrennung, kann aus Epoxibutadien unter den Hydrierbedingungen zur Herstellung von 1,6-Hexandiol im allgemeinen n-Butanol gewonnen werden.

In einer bevorzugten Ausführungsform wird der Metathesekatalysator nach Beendigung der Metathesereaktion vom Reaktionsansatz abgetrennt und rückgeführt, und die Bisepoxihexatriene hydriert. Die Hydrierung wird an einem an sich bekannten Hydrierkatalysator vorgenommen. Es kommen solche Katalysatoren für die Verfahrensstufe b) in Betracht, die die Hydrierung von Olefinen, Ketonen oder Aldehyden mit Wasserstoff zu Kohlenwasserstoffen bzw. Alkoholen ermöglichen. Diese Katalysatoren, wie sie z.B. in Kropf, Methoden der Organischen Chemie, Houben-Weyl, Thieme Verlag 1980, Bd. IV/1c, S. 45 - 66 beschrieben sind, können homogen im Reaktionsansatz vorliegen.

Bevorzugte Hydrierkatalysatoren sind aber heterogene Katalysatoren, wie sie z.B. in Kropf, Methoden der Organischen Chemie, Houben-Weyl, Thieme Verlag 1980, Bd. IV/1c, S. 16 - 44 beschrieben sind. So enthalten die Hydrierkatalysatoren beispielsweise Elemente aus den Gruppen 6 bis 11 des Periodischen Systems der Elemente. Diese können in Form von Metallen, Oxiden oder Sulfiden vorliegen. Sie können beispielsweise als Träger-, Skelett-, Schwarzkatalysatoren oder als metallische Mischkatalysatoren eingesetzt werden. Beispiele sind Pt-Schwarz, Pt/C, Pt/Al₂O₃, PtO₂, Pd-Schwarz, Pd/C, Pd/Al₂O₃, Pd/SiO₂, Pd/CaCO₃, Pd/ BaSO₄, Rh/C, Rh/Al₂O₃, Ru/SiO₂, Ni/SiO₂, Raney-Nickel, Co/SiO₂, Co/Al₂O₃, Raney-Kobalt, Fe, Fe-haltige Mischkatalysatoren, ReSchwarz, Raney-Rhenium, Cu/SiO₂, Cu/Al₂O₃, Raney-Kupfer, Cu/C, PtO₂/Rh₂O₃, Pt/Pd/C, CuCr₂O₄, BaCr₂O₄, Ni/Cr₂O₃/Al₂O₃, Re₂O₇, CoS, NiS, MoS₃, Cu/SiO₂/MoO₃/Al₂O₃. Besonders bevorzugt werden Katalysatoren mit Elementen der Gruppen 7 bis 10 des Periodischen Systems der Elemente wie Pd/C, Pt/C, Re/C, Cu/C, Cu/SiO₂, Ni/C, Raney-Nickel und Raney-Kobalt. Die Katalysatoren können in an sich bekannter Weise vor ihrer Verwendung durch Erhitzen in einer Wasserstoffatmosphäre aktiviert werden.

Die Hydrierung kann kontinuierlich oder diskontinuierlich durchgeführt werden. Der Wasserstoffdruck kann 1 bis 300 bar, bevorzugt 1 bis 100 bar, besonders bevorzugt 1 bis 50 bar betragen bei einer Reaktionstemperatur von 20 bis 300°C, bevorzugt 20 bis 200°C und besonders bevorzugt 20 bis 150°C.

Die Hydrierung der Bisepoxihexatriene kann in Substanz erfolgen oder in einem Lösungsmittel, wobei beispielsweise Ether wie Ethylenglykoldimethylether, Tetrahydrofuran, Dioxan und Diethylether, Alkohole wie Methanol, Ethanol, Propanol und 1,6-Hexandiol, aber auch Wasser in Betracht kommen.

Die Isolierung des erfindungsgemäß hergestellten 1,6-Hexandiols aus dem Reaktionsgemisch kann in an sich bekannter Weise erfolgen, beispielsweise destillativ.

Das erfindungsgemäße Verfahren zur Herstellung von 1,6-Hexandiol aus Epoxibutadien führt, wenn man die Herstellung der jeweiligen Ausgangsverbindungen mitberücksichtigt, in weniger Verfahrensstufen zum gesuchten Endprodukt als die bekannten Verfahren unter Einsatz von Adipinsäure oder deren Derivaten. Weiterhin ist die Hydrierstufe als Niederdruck- oder Mitteldruckverfahren gestaltbar, was im Vergleich zum Stand der Technik verfahrenstechnisch wesentlich einfacher ist und darüberhinaus deutlich geringere Investitionskosten erfordert. Schließlich sind in der Hydrierstufe nur 3 Äquivalente Wasserstoff pro Äquivalent 1,6-Hexandiol erforderlich.

### Beispiele

Das in den Beispielen eingesetzte Epoxibutadien hatte eine Reinheit von ca. 99 %.

Verfahrensstufe a)

### Beispiel 1

20 mg Cl₂(PCy₃)₂Ru=C(Ph)H (Cy = Cyclohexyl, Ph = Phenyl) und 3 g Epoxibutadien wurden unter Argon-Schutzgas 21 h bei 24°C gerührt, wobei freigesetztes Ethen entweichen konnte. Eine gaschromatische Analyse des Reaktionsgemisches ergab einen Umsatz von 3,1 % mit 12 % Selektivität zu den Verbindungen Ia und Ib.

### Beispiel 2

10 mg RuCl₂(p-cumol)PCy₃ (Cy = Cyclohexyl) und 2 g Epoxibutadien wurden in Analogie zu Beispiel 1 23 h bei 23°C gerührt. Die Verfahrensprodukte Ia und Ib wurden bei 3,5 % Umsatz mit 15 % Selektivität erhalten.

### Beispiel 3

10 mg RuCl₂(p-cumol)PCy₃ wurden mit 50 mg Trimethlysilyldiazomethan versetzt, 2 Minuten auf 60°C erhitzt, auf 23°C abgekühlt und mit 2 g Epoxibutadien versetzt. Nach einer Umsetzung in Analogie zu Beispiel 1 wurden nach 19,5 h bei 2,6 % Umsatz die Verfahrensprodukte Ia und Ib mit einer Selektivität von 15 % erhalten.

Verfahrensstufen a) und b)

### Beispiel 4

In Analogie zu Beispiel 1 wurden 20 mg Cl₂(PCy₃)₂Ru=C(Ph)H und 6 g Epoxibutadien umgesetzt. Nach Beendigung der Metathesereaktion wurde von nicht umgesetztem Epoxibutadien abdestilliert und der Rückstand von 0,11 g mit 5 g Tetrahydrofuran und 1,2 g Raney-Kobalt versetzt. Das Gemisch wurde bei 40 bar Wasserstoffdruck und 120°C 2 h hydriert, wobei sich die im Ansatz vorhandenen Bisepoxihexatriene vollständig zu 1,6-Hexandiol umsetzten.

### Beispiel 5

In Analogie zu Beispiel 1 wurden 80 mg Cl₂(PCy₃)₂Ru=C(Ph)H und 11,9 g Epoxibutadien umgesetzt. Die Verfahrensprodukte 1a und 1b wurden bei 3,2 % Umsatz mit 24 % Selektivität erhalten. Vom Reaktionsaustrag wurde nicht umgesetztes Epoxidbutadien destillativ abgetrennt und der Rückstand in 6 g Ethylenglykoldimethylether aufgenommen. Diese Lösung wurde bei 50 bar Wasserstoffdruck bei 100°C 1 h hydriert, wobei sich die im Ansatz vorhandenen Bisepoxihexatriene vollständig zu 1,6-Hexandiol umsetzten.

## Patentansprüche

1. Verfahren zur Herstellung von 1,6-Hexandiol, **dadurch gekennzeichnet, daß** man
a) Epoxibutadien in Gegenwart eines Metathesekatalysators unter Abspaltung von Ethen zu Bisepoxihexatrienen der Formeln Ia und Ib 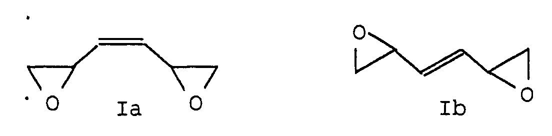 umsetzt und
b) diese Bisepoxihexatriene mit Wasserstoff zu 1,6-Hexandiol hydriert.

2. Verfahren nach Anspruch 1, **dadurch gekennzeichnet, daß** man als Metathesekatalysatoren Rutheniumverbindungen verwendet.

3. Verfahren nach Anspruch 1 oder 2, **dadurch gekennzeichnet, daß** man das während der Reaktion gemäß Verfahrensschritt a) freigesetzte Ethen kontinuierlich entfernt.

4. Verfahren nach den Ansprüchen 1 bis 3, **dadurch gekennzeichnet, daß** man den Verfahrensschritt a) bei einer Temperatur von 15 bis 100°C vornimmt.

5. Verfahren nach den Ansprüchen 1 bis 4, **dadurch gekennzeichnet, daß** man den Verfahrensschritt a) bei einem solchen Druck vornimmt, daß das Epoxibutadien flüssig vorliegt.

6. Verfahren nach den Ansprüchen 1 bis 5, **dadurch gekennzeichnet, daß** man die Hydrierung gemäß Verfahrensschritt b) in Gegenwart des Metathesekatalysators vornimmt.

7. Verfahren nach den Ansprüchen 1 bis 4, **dadurch gekennzeichnet, daß** man die Hydrierung gemäß Verfahrensschritt b) nach Abtrennung des Metathesekatalysators in Gegenwart eines Hydrierkatalysators vornimmt.

8. Verfahren nach den Ansprüchen 1 bis 5, **dadurch gekennzeichnet, daß** man Bisepoxihexatriene der Formeln Ia und Ib 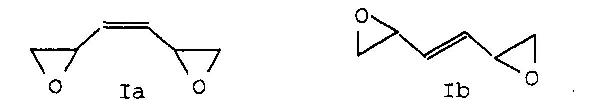 durch Umsetzung von Epoxibutadien in Gegenwart eines Metathesekatalysators unter Abspaltung von Ethen herstellt.

## Claims

1. A process for preparing 1,6-hexanediol, which comprises
a) reacting epoxybutadiene in the presence of a metathesis catalyst with elimination of ethene to give bisepoxyhexatrienes of the formulae Ia and Ib 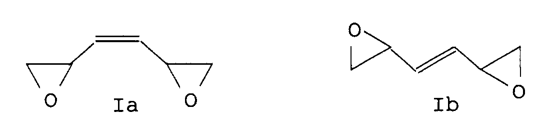 and
b) hydrogenating these bisepoxyhexatrienes with hydrogen to give 1,6-hexanediol.

2. A process as claimed in claim 1, wherein ruthenium compounds are used as metathesis catalyst.

3. A process as claimed in claim 1 or 2, wherein the ethene liberated during the reaction according to process step a) is removed continuously.

4. A process as claimed in any of claims 1 to 3, wherein the process step a) is carried out at from 15 to 100°C.

5. A process as claimed in any of claims 1 to 4, wherein the process step a) is carried out at a pressure at which the epoxybutadiene is present in liquid form.

6. A process as claimed in any of claims 1 to 5, wherein the hydrogenation according to process step b) is carried out in the presence of the metathesis catalyst.

7. A process as claimed in any of claims 1 to 4, wherein the hydrogenation according to process step b) is carried out after the separation of the metathesis catalyst in the presence of a hydrogenation catalyst.

8. A process as claimed in any of claims 1 to 5, wherein bisepoxyhexatrienes of the formulae Ia and Ib 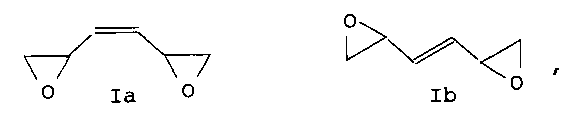 are prepared by reacting epoxybutadiene in the presence of a metathesis catalyst with elimination of ethene.

## Revendications

1. Procédé de préparation de 1,6-hexanediol, **caractérisé en ce que**
a) on fait réagir de l'époxybutadiène, en présence d'un catalyseur de métathèse, avec dissociation de l'éthène jusqu'à obtenir des bisépoxyhexatriènes de formules Ia et Ib 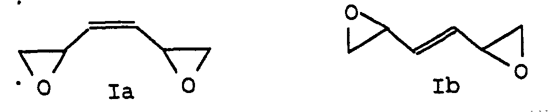 et
b) on hydrogène ces bisépoxyhexatriènes, avec de l'hydrogène, pour obtenir le 1,6-hexanediol.

2. Procédé selon la revendication 1, **caractérisé en ce qu'**on utilise en tant que catalyseurs de métathèse des composés du ruthénium.

3. Procédé selon la revendication 1 ou 2, **caractérisé en ce qu'**on élimine en continu l'éthène libéré au cours de la réaction selon l'étape a).

4. Procédé selon les revendications 1 à 3, **caractérisé en ce qu'**on met en oeuvre l'étape a) à une température de 15 à 100°C.

5. Procédé selon les revendications 1 à 4, **caractérisé en ce qu'**on met en oeuvre l'étape a) sous une pression sous laquelle l'époxybutadiène est présent à l'état liquide.

6. Procédé selon les revendications 1 à 5, **caractérisé en ce qu'**on met en oeuvre l'hydrogénation selon l'étape b) en présence du catalyseur de métathèse.

7. Procédé selon les revendications 1 à 4, **caractérisé en ce qu'**on met en oeuvre l'hydrogénation selon l'étape b) après séparation du catalyseur de métathèse en présence d'un catalyseur d'hydrogénation.

8. Procédé selon les revendications 1 à 5, **caractérisé en ce qu'**on prépare des bisépoxyhexatriènes ayant les formules Ia et Ib 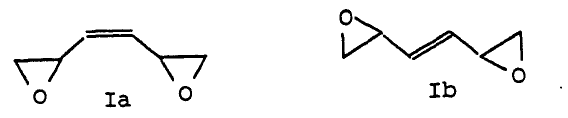 par réaction d'époxybutadiène en présence d'un catalyseur de métathèse, avec dissociation de l'éthène.
